(19) Europäisches Patentamt
European Patent Office
Office européen des brevets

(11) EP 0 976 823 A1

(12) **EUROPÄISCHE PATENTANMELDUNG**

(43) Veröffentlichungstag:
**02.02.2000 Patentblatt 2000/05**

(21) Anmeldenummer: **98113876.1**

(22) Anmeldetag: **22.07.1998**

(51) Int Cl.[7]: **C12N 15/12**, C12N 15/53, C12N 15/55, C12N 15/62, C12N 15/70, C12N 15/85, C12N 9/16, C12N 9/02, C07K 14/47, G01N 33/50, A61K 38/00

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **Völkel, Helge, Dr.**
**89081 Ulm (DE)**

(72) Erfinder: **Völkel, Helge, Dr.**
**Albert-Einstein-Allee 11, 89081 Ulm (DE)**

(74) Vertreter: **Patentanwälte**
**Ruff, Beier, Schöndorf und Mütschele**
**Willy-Brandt-Strasse 28**
**70173 Stuttgart (DE)**

Bemerkungen:
The applicant has subsequently filed a sequence listing and declared, that it includes no new matter.

(54) **Rekombinantes Expressions System und Hoch-Durchsatz BioAssay für die therapeutische Anwendung von Calcineurin-A-Alpha, Calcineurin-A-Beta, Calcineurin-A-Gamma, Calcineurin-B und Copper/Zinc-Superoxide Dismutase bzw. zur Identifizierung von Pharmazeutika**

(57) Molekulare und zelluläre Bioassays dienen der Identifizierung von Substabzen, welche die stabilisierende Wechselwirkung zwischen Calcineurin und CuZnSOD stören bzw. welche direkt an der Genregulation beider Enzyme beteiligt sind. Als messbare Targets für die Analyse durch Laser-Korrelations-Spektroskopie dienen fluoreszenzmarkierte CuZnSOD (gekoppelter chemischer Farbstoff oder EGFP Fusionsprotein) bzw. Calcineurin Isoenzyme (EGFP Fusionsprotein) oder ein neu konstruiertes Peptidsubstrat (RII-Fluophos).

Inhibitoren oder Aktivatoren der Calcineurin oder CuZnSOD sind potentielle Pharmazeutika zur Behandlung neurologischer Erkrankungen, Immunerkrankungen und Herz-Kreislauf-Erkrankungen.

Die CuZnSOD selber soll als Therapeutikum zur Behandlung neurologischer Erkrankungen, Immunerkrankungen und Herz-Kreislauf-Erkrankungen eingesetzt werden. Weiterhin wird die Verwendung der CuZnSOD als Kosmetikum, Antialterungs- und Regeneration-Therapeutikum beansprucht.

Die Histidin getaggten CuZnSOD bzw. Calcineurin Fusionproteine sollen darüber hinaus zur Isolierung von CuZnSOD bzw. Calcineurin Liganden verwendet werden.

EP 0 976 823 A1

## Beschreibung

**Details:** Durch den Bioassay wird die Interaktion rekombinant exprimierter humaner CuZnSOD mit humanen Calcineurin Isoformen (A-alpha, A-beta, A-Gamma, B) in Gegenwart von Calcium und Calmodulin quantifiziert. Auf biochemischer Ebene dient das Heterotetramer Calcineurin-A*, Calcineurin-B, Calmodulin, CuZnSOD als Target für Substanzen, die diesen Komplex stabilisieren oder zerstören können. Gemessen wird die Bindungskonstante zwischen Calcineurin-A und CuZnSOD durch Fluoreszenzdetektion. Die CuZnSOD ist dabei mit einem Fluoreszenzfarbstoff markiert. Meßprinzip ist die Laser-Korrelations-Spektroskopie. Alternativ oder in Kombination mit CuZnSOD kann ein fuorogenes Peptid** (RII-Fluophos) als Meßsubstrat benutzt werden. Das beschriebene Meßprinzip kommt dabei grundsätzlich in zwei Testsystemen zur Anwendung:

1. Einem molekularen in vitro Bioassay (proteinchemisch isoliertes Protein-Heteroteramer in Kombination mit dem fluorogenem Peptid RII-FluoPhos)
2. Einem zellulären in vivo Bioassay (mit Calcineurin-A, Calcineurin-B und fluoreszierender CuZnSOD bzw. RII-Fluophos Peptid transfizierte Säugerzellen).

**Ziel:** Identifizierung von Inhibitoren und Aktivatoren des Signalenzymes Calcineurin. Inhibitoren und Aktivatoren des Calcineurins sind als potentielle Pharmazeutika für die Therapie chronischer und akuter Neurodegeneration, entzündlicher Erkrankungen, Herz-Kreislauf Erkrankungen, zur Immunsuppression bei der Transplantationschirurgie.

**Inhibitoren:** Als Inhibitoren werden Substanzen definiert,

a) die im molekularen **oder** zellulären Bioassay die Interaktion von Calcineurin-A mit CuZnSOD vermindern (da die CuZnSOD selber ein physiologischer Aktivator ist)
b) die im molekularen **oder** zellulären Bioassay die Interaktion mit dem RII-Fluophos Peptid vermindern
c) die im zellulären Bioassay die Proteinexpression der aktivierenden CuZnSOD auf transkriptionaler, translatorischer und/oder posttranslationaler Ebene vermindern.
d) die im zellulären Bioassay die Proteinexpression von Calcineurin-A Isoformen oder Calcineurin-B auf transkriptionaler, translatorischer und/oder posttranslationaler Ebene vermindern.

**Aktivatoren:** Als Aktivatoren werden Substanzen definiert, welche die gegenteiligen Effekte wie unter "Inhibitoren" Punkte a) - d) beschrieben zeigen.

## Zeichnungen/Sequenzen/Protokolle

**1. Prokaryontische DNA Expressionsvektoren (siehe beiligende Sequenzen)**

1.1. DNA Vektor CuZnSOD-pQE30
1.2. DNA Vektor CuZnSOD-pQE60
1.3. DNA Vektor CNAa1-pQE30
1.4. DNA Vektor CNAa2-pQE30
1.5. DNA Vektor CNAb1-pQE30
1.6. DNA Vektor CNAb2-pQE30
1.7. DNA Vektor CNAg1-pQE30
1.8. DNA Vektor CNAg2-pQE30

**2. Eukaryontische DNA Expressionsvektoren (siehe beiligende Sequenzen)**

2.1. DNA Vektor CuZnSOD-pEGFP
2.2. DNA Vektor CNAa-pEGFP
2.3. DNA Vektor CNAb-pEGFP
2.4. DNA Vektor CNAg-pEGFP

**3. RII-Fluophos-Peptid**

• im zellulären und molekularen Bioassay sollen die Calcineurin Isoformen A-Alpha, A-Beta und A-Gamma getrennt und vergleichend untersucht werden.
** siehe Zeichnung, Sequenzen, Formeln

# RII-FluoPhos (Alle Variationen aus R1, R2, R3)

R1 = H

R2= 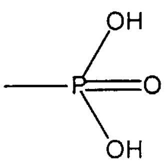

R3 = 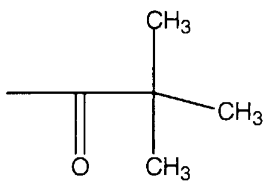

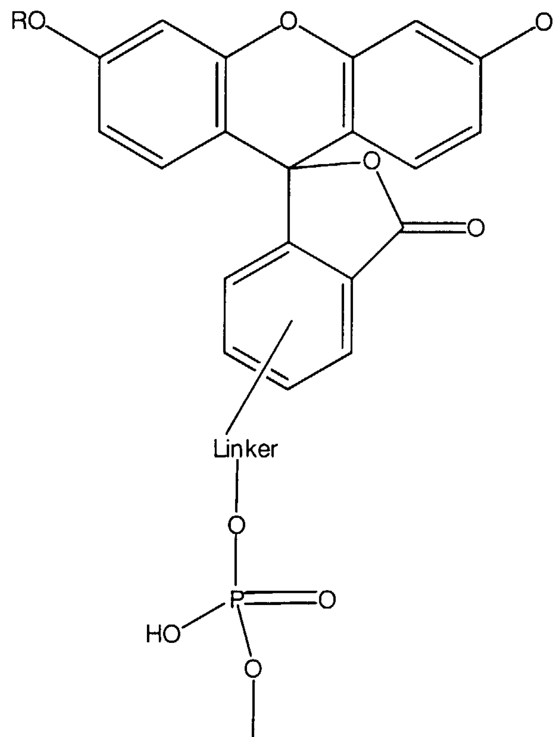

-NH2-Asp-Leu-Asp-Val-Pro-Ile-Pro-Gly-Arg-Phe-Asp-Arg-Arg-Val-Ser-Val-Ala-Ala-Glu-COOH

- Synthese aus Apopeptid mittels Fluorescein-phosphoramiditen (Serin-OH Kopplung an Farbstoffphosphoramidite)
- Enzymatische Phosphorylierung (R2) der Fluoresceinseitenketten mittels ATP und Tyrosinkinase

**4. Molekulare Bioassays auf der Basis der Koexpression von Calcineurin-A, Calcineurin-B und CuZnSOD**

Calcineurin-A (CNA)
Calcineurin-B (CNB)
Calmodulin (CaM)
Kupfer/Zink-Superoxidedismutase (SOD)
RII-Fluophos Substrat (RII*)
Fluoreszenzfarbstoff (*)
(...) = Komplex
- = Bindung

Aktivator

4.1 CNA + CNB + CaM + SOD* ⇄ (CNA-CNB-CaM+SOD*)

Inhibitor

4.2 CNA +CNB + CaM + RII*+SOD* ⇄ (CNA-CNB-CaM-RII*-SOD*)+Phosphat

(→ Aktivator; ← Inhibitor)

5. Zelluläre Bioassays auf der Basis der Expression eines fluoreszierenden Reporterproteins (Calcineurin-A oder CuZnSOD).

5.1 Zelle mit homolog rekombiniertem fluoreszierenden Calcineurin Fusionsgen

CNA*-Gen → CNA*-messenger RNA → CNA*-Protein

↓ mögliche Regulationsebenen von Calcineurin Modulatoren (Transkription, RNA-Prozessing, Translation, postranslationale Modifikation)
Aktivator: CNA*-Protein Konzentration steigt
Inhibitor: CNA*-Protein Konzentration fällt

5.2 Zelle mit homolog rekombiniertem fluoreszierenden Calcineurin Fusionsgen

CNA*-Gen → CNA*-messenger RNA → CNA*-Protein

↓ mögliche Regulationsebenen von CuZnSOD Modulatoren (Transkription, RNA-Prozessing, Translation, postranslationale Modifikation)
Aktivator: CUZnSOD*-Protein Konzentration steigt (Calcineurin stabilisiert)
Inhibitor: CUZnSOD*-Protein Konzentration fällt (Calcineurin destabilisiert)

**6. Präparative Affinitätschromatographie mit rekombinanten His-Tag-CuZnSOD/-Calcineurin**
NTA = Nitrilotriacetat

NiNTA (bzw. Cu/ZnNTA)-Matrix (Chromatographiematerial, Biosensor, etc.)

His-Tag-CuZnSOD → CuZnSOD Liganden

His-Tag-Calcineurin → Calcineurin Liganden

Liganden = niedermolekulare endogene, exogene und synthetische Stoffe, wechselwirkende Makromoleküle wie Peptide, Proteine, Kohkenhydrate, Lipide, Nukleinsäuren, synthetische Polymere

## Patentansprüche

**Die Patentansprüche umfaßen:**

1. Prokaryontische DNA Expressionsvektoren für die rekombinante Expression von Calcineurin-A, Calcineurin-B und CuZnSOD (molekularer Assay)
2. Eukaryontische DNA Expressionsvektoren für die Transfektion von Säugerzellen mit Calcineurin-A, Calcineurin-B und CuZnSOD (zellulärer Assay)
3. Reinigungs- und Prozessierungsprotokolle für rekombinante Calcineurin und CuZnSOD Proteine
4. Verwendung des RII-Fluophos-Peptid als Phosphatase Ligand
5. Bioassay auf der Basis der Koexpression von Calcineurin-A, Calcineurin-B und CuZnSOD
6. Therapieanwendung von humaner rekombinanter CuZnSOD
7. Therapieanwendung von Calcineurin Inhibitoren und Aktivatoren
8. Präparative Affinitätschromatographie und Biosensoranwendungen auf der Basis rekombinanter CuZnSOD bzw Calcineurine.

**1. Prokaryontische DNA Expressionsvektoren für die rekombinante Expression von Calcineurin-A, Calcineurin-B und CuZnSOD (molekularer Assay)**

1.1 DNA Vektor CuZnSOD-pQE30
1.2. DNA Vektor CuZnSOD-pQE60
1.3 DNA Vektor CNAa1-pQE30
1.4 DNA Vektor CNAa2-pQE30
1.5 DNA Vektor CNAb1-pQE30
1.6 DNA Vektor CNAb2-pQE30
1.7 DNA Vektor CNAg1-pQE30
1.8 DNA Vektor CNAg2-pQE30

**2. Eukaryontische DNA Expressionsvektoren für die Transfektion von Säugerzellen mit Calcineurin-A, Calcineurin-B und CuZnSOD (zellulärer Assay)**

2.1 DNA Vektor CuZnSOD-pEGFP
2.2 DNA Vektor CNAa-pEGFP
2.3 DNA Vektor CANb-pEGFP
2.4 DNA Vektor CNAg-pEGFP

**3. Reinigungs- und Prozessierungsprotokolle für rekombinante Calcineurin und CuZnSOD Proteine**

3.1 Reinigung von Calcineurin A und B mittels einer Histidin-Tag-CuZnSODAffinitätschromatographie (Rekombinante CuZnSOD ist nichtkovalent über einen Histidin-Tag an Kupfer-Zink-Nitrilotriacetat gekoppelt und dient seinerseits als Affinitätsligand für den Calcineurin-A/B Komplex.
3.2 Reinigung anderer Kupfer oder/und Zink bindender Proteine mittels Cu/Zn-Nitriloacetat.
3.3 Reinigung anderer CuZnSOD Liganden (endogener oder exogener) mittels Histidin-Tagged-CuZnSOD-Protein.
3.4 N- und C-terminale Prozessierung von Polyhistidin-getaggter-CuZnSOD bzw. Polyhistidin-tag Calcineurin mit Cathepsin-C bzw. Carboxypeptidase-A.

**4. RII-Fluophos-Peptid**

4.1 Verwendung als Phosphatase Enzymsubstrat oder Ligand für molekularbiologische, biochemische oder zellbiologische Assays.
4.2 Verwendung als Peptid-Marker in der Fluoreszensmikroskopie.
4.3 Verwendung von Tyrosinkinasen zur Seitenkettenphosphorylierung von Fluorescein und andern Farbstoffen.

**5. Bioassay auf der Basis der Koexpression von Calcineurin-A, Calcineurin-B und CuZnSOD**

5.1 Verwendung zur Identifikation von Calcineurin-A Liganden.
5.2 Verwendung zur Identifikation von Calcineurin-B Liganden.
5.3 Verwendung zur Identifikation von CuZnSOD Liganden.
5.4 Etablierung von high-Throughput Testsystemen mit Calcineurin auf der Basis von rekombinanter CuZnSOD bzw. RII-Fluophos Peptid.

**6. Therapieanwendung von humaner rekombinanter CuZnSOD**
Verwendung als topisch, oral, intramuskulär, intravenös, intrathekal oder intracerebroventrikulär appliziertes Therapeutikum zur Behandlung von:

6.1 Reperfusionsschäden in der Transplantationschirurgie, Ischämie und Hypoxie des Herzens bzw. Zentralen Nervensystems.
6.2 Entzündlichen Erkrankungen, insbesondere des Nervensystems, des Verdauungstraktes, der Blutgefäße und viraler bzw. bakteriologischer Infektionen.
6.3 Behandlung akuter und chronischer neurodegenerativer Erkrankungen (Schlaganfall, Epilepsie, Alzheimer, Parkinson, ALS).
6.4 Behandlung akuter und chronischer Alterungsprozesse.
6.5 Verwendung als Kosmetikum und Regerationstherapeutikum bei Verbrennungen und anderen offenen Wunden.

**7. Therapieanwendung von Calcineurin Inhibitoren und Aktivatoren**

7.1 Behandlung von akuter und chronischer Neurodegeneration, insbesondere Schlaganfall, Epilepsie, Alzheimer, Parkinson und ALS.
7.2 Verwendung als Immunsuppressiva bei entzündlicher Erkrankungen oder in der Transplantationschirurgie.
7.3 Verwendung als Herz-/-Kreislauftherapeutikum, insbesondere zur Therapie bei Herzinfarkt und Herzhypertrophie.

**8. Präparative Affinitätschromatographie und Biosensoranwendungen auf der Basis rekombinanter CuZnSOD bzw Calcineurine**

8.1 Präparative Isolierung von CuZnSOD Liganden mittels rekombinanter CuZnSOD als Affinitätsligand

8.2 Präparative Isolierung von Calcineurin Liganden mittels rekombinanten Calcineurinen als Affinitätsliganden
8.3 Verwendung rekombinanter CuZnSOD als Biosensorligand
8.4 Verwendung rekombinanter calcineurine auf Biosensorligand

Europäisches Patentamt

# EUROPÄISCHER TEILRECHERCHENBERICHT

der nach Regel 45 des Europäischen Patent-übereinkommens für das weitere Verfahren als europäischer Recherchenbericht gilt

Nummer der Anmeldung
EP 98 11 3876

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | DATABASE WPI<br>Section Ch, Week 9431<br>Derwent Publications Ltd., London, GB;<br>Class B04, AN 94-251696<br>XP002098865<br>& JP 06 181778 A (TAKEDA CHEM IND LTD)<br>, 5. Juli 1994<br>* Zusammenfassung *<br>--- | 1 | C12N15/12<br>C12N15/53<br>C12N15/55<br>C12N15/62<br>C12N15/70<br>C12N15/85<br>C12N9/16<br>C12N9/02<br>C07K14/47<br>G01N33/50<br>A61K38/00 |
| X | T. MURAMATSU AND R.L. KINCAID: "Molecular cloning of a full-length cDNA encoding the catalytic subunit of human calmodulin-dependent protein phosphatase (Calcineurin A alpha)"<br>BIOCHIMICA BIOPHYSICA ACTA,<br>Bd. 1178, 1993, Seiten 117-120,<br>XP002098863<br>ELSEVIER SCIENCE, AMSTERDAM, NL<br>* das ganze Dokument *<br>---<br>-/-- | 1 | |
| | | | RECHERCHIERTE SACHGEBIETE (Int.Cl.6) |
| | | | C12N<br>C07K<br>G01N<br>A61K |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

1.3

Grund für die Beschränkung der Recherche:

Der Inhalt von Anspruch 1.3, der sich auf einen prokaryontischen Expressionsvektor bezieht und den DNA Vektor CNAa1-pQE30 beinhaltet, konnte nicht gesucht werden, da dieser Vektor nicht ausreichend offenbart wurde.

| Recherchenort | Abschlußdatum der Recherche | Prüfer |
|---|---|---|
| DEN HAAG | 6. April 1999 | HORNIG H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches
Patentamt

Nummer der Anmeldung
EP 98 11 3876

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-teilweise, 1.3-vollständig

Europäisches Patentamt

**EUROPÄISCHER TEILRECHERCHENBERICHT**

Nummer der Anmeldung
EP 98 11 3876

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | R.L. KINCAID ET AL.: "Cloning and characterization of molecular isoforms of the catalytic subunit of calcineurin using nonisotopic methods"<br>J. BIOL. CHEM.,<br>Bd. 265, Nr. 19, 5. Juli 1990, Seiten 11312-11319, XP002098864<br>AM. SOC. BIOCHEM. MOL.BIOL.,INC.,BALTIMORE,US<br>* das ganze Dokument *<br>----- | 1 | |
| | | | RECHERCHIERTE SACHGEBIETE (Int.Cl.6) |

EPO FORM 1503 03.82 (P04C12)

Europäisches Patentamt

**MANGELNDE EINHEITLICHKEIT DER ERFINDUNG ERGÄNZUNGSBLATT B**

Nummer der Anmeldung
EP 98 11 3876

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-teilweise, 1.3-vollständig

   Prokaryontischer DNA Expressionsvektor für die rekombinante Expression von Calcineurin-A; besagter Expressionsvektor, wobei dieser DNA Vektor CNAa1-pQE30 ist;

2. Ansprüche: 1-teilweise, 1.4-vollständig

   Prokaryontischer DNA Expressionsvektor für die rekombinante Expression von Calcineurin-A; besagter Expressionsvektor, wobei dieser DNA Vektor CNAa2-pQE30 ist;

3. Ansprüche: 1-teilweise, 1.5-vollständig

   Prokaryontischer DNA Expressionsvektor für die rekombinante Expression von Calcineurin-A; besagter Expressionsvektor, wobei dieser DNA Vektor CNAb1-pQE30 ist;

4. Ansprüche: 1-teilweise, 1.6-vollständig

   Prokaryontischer DNA Expressionsvektor für die rekombinante Expression von Calcineurin-A; besagter Expressionsvektor, wobei dieser DNA Vektor CNAb2-pQE30 ist;

5. Ansprüche: 1-teilweise, 1.7-vollständig

   Prokaryontischer DNA Expressionsvektor für die rekombinante Expression von Calcineurin-A; besagter Expressionsvektor, wobei dieser DNA Vektor CNAg1-pQE30 ist;

6. Ansprüche: 1-teilweise, 1.8-vollständig

   Prokaryontischer DNA Expressionsvektor für die rekombinante Expression von Calcineurin-A; besagter Expressionsvektor, wobei dieser DNA Vektor CNAg2-pQE30 ist;

7. Ansprüche: 1-teilweise

   Prokaryontischer DNA Expressionsvektor für die rekombinante Expression von Calcineurin-B;

8. Ansprüche: 1-teilweise, 1.1-vollständig

   Prokaryontischer DNA Expressionsvektor für die rekombinante Expression von CuZnSOD; besagter Expressionsvektor, wobei

Europäisches Patentamt

**MANGELNDE EINHEITLICHKEIT DER ERFINDUNG ERGÄNZUNGSBLATT B**

Nummer der Anmeldung
EP 98 11 3876

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

dieser DNA Vektor CuZnSOD-pQE30 ist;

9. Ansprüche: 1-teilweise, 1.2-vollständig

Prokaryontischer DNA Expressionsvektor für die rekombinante Expression von CuZnSOD; besagter Expressionsvektor, wobei dieser DNA Vektor CuZnSOD-pQE60 ist;

10. Ansprüche: 2-teilweise, (2.2, 2.3, 2.4)-vollständig

Eukaryontische DNA Expressionsvektoren für die Transfektion von Säugezellen mit Calcineurin-A; besagte Expressionsvektoren, wobei die DNA Vektoren CNAa-pEGFP, CNAb-pEGFP und CNAg-pEFG umfasst;

11. Ansprüche: 2-teilweise, 2.1-vollständig

Eukaryontischer DNA Expressionsvektor für die Transfektion von Säugezellen mit CuZnSOD; besagter Expressionsvektor, wobei der DNA Vektor CuZnSOD-pEGFP umfasst;

12. Ansprüche: (3, 3.4)-teilweise, 3.1-vollständig

Reinigungs- und Prozessierungsprotokolle für das rekombinante Calcineurin Protein; Reinigung von Calcineurin A und B mittels einer Histidin-Tag-CuZnSOD Affinitätschromatographie (Rekombinante CuZnSOD ist nicht kovalent über einen Histidin-Tag an Kupfer-Zink-Nitrilotriacetat gekoppelt und dient seinerseits als Affinitätsligand für den Calcineurin-A/B Komplex; N- und C-terminale Prozessierung von Polyhistidin-getaggtem-Calcineurin mit Cathepsin-C bzw. Carboxypeptidase-A;

13. Ansprüche: (3, 3.4)-teilweise

Reinigungs- und Prozessierungsprotokolle für das rekombinante CuZnSOD Protein; Reinigung von CuZnSOD mittels einer Kupfer-Zink-Nitriloactetat-Affinitätschromatographie (Rekombinante CuZnSOD wird nicht kovalent über einen Histidin-Tag an Kupfer-Zink-Nitrilotriacetat gekoppelt); N- und C-terminale Prozessierung von Polyhistidin-getaggter-CuZnSOD mit Cathepsin-C bzw. Carboxypeptidase-A;

14. Ansprüche: 3.2-vollständig

Reinigung anderer Kupfer oder/und Zink bindender Proteine

Europäisches Patentamt

**MANGELNDE EINHEITLICHKEIT DER ERFINDUNG ERGÄNZUNGSBLATT B**

Nummer der Anmeldung
EP 98 11 3876

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

mittels Cu/Zn-Nitriloacetat;

15. Ansprüche: 3.3-vollständig

    Reinigung anderer CuZnSOD Liganden (endogener oder exogener) mittels Histidin-Tagged-CuZnSOD-Protein;

16. Ansprüche: 4-teilweise, 4.1-vollständig

    Verwendung des RII-Fluophos-Peptid als Phosphatase Ligand; Verwendung als Phosphatase Enzymsubstrat oder Ligand für molekularbiologische, biochemische oder zellbiologische Assays;

17. Ansprüche: 4-teilweise, 4.2, 4.3-vollständig

    Verwendung des RII-Fluophos-Peptid als Phosphatase Ligand; Verwendung als Peptid-Marker in der Fluoreszenzmikroskopie; Verwendung von Tyrosinkinasen zur Seitenkettenphosphorylierung von Fluorescein und anderen Fabstoffen;

18. Ansprüche: (5, 5.1, 5.2, 5.3, 5.4)-vollständig

    Bioassay auf der Basis der Koexpression von Calcineurin-A, Calcineurin-B und CuZnSOD; Verwendung zur Identifikation von Calcineurin-A-Liganden; Verwendung zur Identifikation von Calcineurin-B-Liganden; Verwendung zur Identifikation von CuZnSOD-Liganden; Etablieruntg von high-Throughput Testsystemen mit Calcineurin auf der Basis von rekombinanter CuZnSOD bzw. RII-Fluophos Peptid;

19. Ansprüche: 6-teilweise, 6.1-vollständig

    Therapieanwendung von humaner rekombinanter CuZnSOD; Verwendung als topisch oral, intramuskulär, intravenös, intrathekal oder intracerebroventrikulär appliziertes Therapeutikum zur Behandlung von: Reperfusionsschäden in der Transplantationschirurgie, Ischämie und Hyperoxie des Herzens bzw. Zentralen Nervensystem;

20. Ansprüche: 6-teilweise, 6.2-vollständig

    Therapieanwendung von humaner rekombinanter CuZnSOD; Verwendung als topisch oral, intramuskulär, intravenös, intrathekal oder intracerebroventrikulär appliziertes Therapeutikum zur Behandlung von: Entzündlichen

Europäisches Patentamt

**MANGELNDE EINHEITLICHKEIT DER ERFINDUNG ERGÄNZUNGSBLATT B**

Nummer der Anmeldung
EP 98 11 3876

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Erkrankungen, insbesondere des Nervensystems, des Verdauungstraktes, der Blutgefässe und viraler bzw. bakteriologischer Infektionen;

21. Ansprüche: 6-teilweise, 6.2-vollständig

Therapieanwendung von humaner rekombinanter CuZnSOD; Verwendung als topisch oral, intramuskulär, intravenös, intrathekal oder intracerebroventrikulär appliziertes Therapeutikum zur Behandlung von akuten und chronischen neurodegenerativen Erkrankungen (Schlaganfall, Epilepsie, Alzheimer, Parkinson, ALS);

22. Ansprüche: 6-teilweise, 6.4-vollständig

Therapieanwendung von humaner rekombinanter CuZnSOD; Verwendung als topisch oral, intramuskulär, intravenös, intrathekal oder intracerebroventrikulär appliziertes Therapeutikum zur Behandlung akuter und chronischer Alterungsprozesse;

23. Ansprüche: 6-teilweise, 6.5-vollständig

Therapieanwendung von humaner rekombinanter CuZnSOD; Verwendung als topisch oral, intramuskulär, intravenös, intrathekal oder intracerebroventrikulär appliziertes Therapeutikum zur Verwendung als Kosmetikum und Regerationstherapeutikum bei Verbrennungen und anderen offenen Wunden;

24. Ansprüche: 7-teilweise, 7.1-vollständig

Therapieanwendungen von Calcineurin Inhibitoren und Aktivatoren; Behandlung von akuter und chronischer Neurodegeneration, insbesondere Schlaganfall, Epilepsie, Alzheimer, Parkinson und ALS;

25. Ansprüche: 7-teilweise, 7.2-vollständig

Therapieanwendungen von Calcineurin Inhibitoren und Aktivatoren; Verwendung als Immunosuppressiva bei entzündlichen Erkrankungen oder in der Transplantationschirurgie;

26. Ansprüche: 7-teilweise, 7.2-vollständig

Europäisches Patentamt

**MANGELNDE EINHEITLICHKEIT DER ERFINDUNG ERGÄNZUNGSBLATT B**

Nummer der Anmeldung
EP 98 11 3876

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Therapieanwendungen von Calcineurin Inhibitoren und Aktivatoren; Verwendung als Herz-/-Kreislauftherapeutikum, insbesondere zur Therapie bei Herzinfarkt und Herzhypertrophie;

27. Ansprüche: 8-teilweise, (8.1, 8.3)-vollständig

Präparative Affinitätschromatographie und Biosensoranwendungen auf der Basis rekombinanter CuZNSOD; Präparative Isolierung von CuZnSOD Liganden mittels rekombinanter CuZnSOD als Affinitätsligand; Verwendung rekombinanter CuZnSOD als Biosensorligand;

28. Ansprüche: 8-teilweise, (8.2, 8.4)-vollständig

Präparative Affinitätschromatographie und Biosensoranwendungen auf der Basis rekombinantem Calcineurin; Präparative Isolierung von Calcineurin Liganden mittels rekombinanter Calcineurinen als Affinitätsligand; Verwendung rekombinanter Calcineurine auf Biosensorligand;

## Annex to the description

SEQUENZPROTOKOLL

```
<110> Völkel, Helge
      Universität Ulm - Klinikum

<120> Sequenzprotokoll zu Abbildung 2 (Aminosäure), ABB2:
      RII-Fluophos

<130> VOLKEL-ULM-2-0798

<140> 98113876.1-2106
<141> 1999-01-02

<150> 98113876.1-2106
<151> 1998-07-22

<160> 1

<170> PatentIn Ver. 2.1

<210> 1
<211> 19
<212> PRT
<213> Bos taurus

<220>
<221> CHAIN
<222> (1)..(19)
<223> peptide of cAMP regulated proteinkinase A (bovine)

<220>
<221> MOD_RES
<222> (15)
<223> PHOSPHORYLATION  by proteinkinases,
      dephosphorylation by calcineurin and other
      phosphoproteinphosphatases

<220>
<221> DOMAIN
<222> (1)..(19)
<223> Binding to Calcineurin, neccessary for efficient
      phosphorylation

<220>
<221> SITE
<222> (15)
<223> ARTIFICIAL CHEMICAL MODIFICATION, Diphosphoester
      to Ser-15 and fluoresceine derivates. 3´ and 6´
```

```
       hydroxy groups of fluoresceine may be
       phosphorylated enyzmatically

<300>
<301> Blumenthal, Donald K.
      et, al.
<302> Dephosphorylation of cAMP-dependent protein kinase
      regulatory subunit (type II) by calmodulin-dependent
      protein phosphatase
<303> J. Biol. Chem.
<304> 18
<305> 261
<307> 1986-06-25

<400> 1
Asp Leu Asp Val Pro Ile Pro Gly Arg Phe Asp Arg Arg Val Ser Val
 1               5                   10                  15

Ala Ala Glu
```